# EUROPEAN PATENT SPECIFICATION

(11) **EP 2 376 912 B1**
(45) Date of publication and mention of the grant of the patent: **17.04.2013**
(21) Application number: 10701559.6
(22) Date of filing: 07.01.2010
(51) Int. Cl.: G01N 33/49, G01N 33/487

(54) **METHOD AND APPARATUS FOR ALCOHOL CONCENTRATION DETECTION IN A BLOOD SAMPLE**
VERFAHREN UND VORRICHTUNG ZUM NACHWEIS DER ALKOHOLKONZENTRATION IN EINER BLUTPROBE
PROCÉDÉ ET APPAREIL PERMETTANT LA DÉTERMINATION DE L'ALCOOLÉMIE À PARTIR D'UN ÉCHANTILLON DE SANG

(30) Priority: 08.01.2009 GB 0900176
(43) Date of publication of application: 19.10.2011
(73) Proprietor: Campbell, James Gordon, Derby DE1 3QB (GB); Touquet, Robin, London SW9 6DA (GB)
(72) Inventor: Campbell, James Gordon, Derby DE1 3QB (GB); Touquet, Robin, London SW9 6DA (GB)
(74) Representative: Swindell & Pearson Limited
(86) International application number: PCT/GB2010/000018
(87) International publication number: WO 2010/079329

(56) References cited:
- EP-A1- 1 326 075
- GB-A- 1 394 171
- US-A- 6 123 828

## Description

### FIELD OF INVENTION

The present invention relates to a rapid diagnostic device for testing blood to determine the concentration of blood ethyl alcohol in the sample using a fuel cell.

The detection system comprises a swab carrier capable of receiving the blood sample via a capillary which isolates the sample in a test chamber within the instrument. The instrument then conditions the sample so that that the equilibrium vapour pressure of the ethyl alcohol is generated quickly and accurately in the head space according to universal gas laws relating to partial pressure. The concentration of the ethyl alcohol vapour at that equilibrium temperature is then measured using a fuel cell by taking into the cell a measured volume from the head space. The blood alcohol content is calculated according to reference to a calibration curve and is read visually or incorporated into a data logging system. The detection system is suitable for use in all circumstances where the rapid detection of blood alcohol is desirable or necessary, having a detection level suitable for detection of alcohol at all typical levels in human whole blood, including serious cases of alcohol poisoning where speed of result is paramount. It is also suitable for detecting alcohol in other bodily fluids for example plasma, serum or saliva.

For convenience the apparatus may be used for, but is not restricted to, a small volume of blood such as 30 microlitres via a fingerprick lancet device. There are convincing reasons why blood alcohol is better than other methodologies such as breath, saliva, lacrimal fluid, and the like.

### BACKGROUND TO THE INVENTION

Alcohol is the most common drug of abuse and it is employed almost universally worldwide recreationally, frequently to excess and it is often abused and consumed in quantities that cause inebriation, intoxication, and loss of control.

It is frequently associated with injuries requiring a hospital visit.

It is now accepted that early identification of alcohol misuse in the ED with the giving of Brief Advice (BA), together with the offer of an appointment with an Alcohol Nurse Specialist (ANS) for Brief Intervention (BI) results in reduced ED reattendance within the next 12 months (Crawford et al 2004; Ahmed & Mackway-Jones 2007). For the patient who is alert and orientated taking a history - or using a specific questionnaire such as the Paddington Alcohol Test (PAT) - is the best form of detection. But this is not possible for the patient with a diminished level of consciousness for what ever reason including alcohol misuse, i.e. for the obtunded patient. By having a simple, robust and immediate system of determining Blood Alcohol Concentration (BAC) the likelihood of accurate clinical assessment at the start of care will be improved (both for raised levels which may explain the obtunded clinical state, and where a low level definitely excludes alcohol as a cause of such). This identification should lead to feed-back when sober - perhaps by applying PAT - so as to make best use of the 'opportunistic teachable moment' (Williams S, Brown A, Patton R, Crawford MJ, Touquet R. The half-life of the 'teachable moment' for alcohol misusing patients in the emergency department. Drug and Alcohol Dependence 2005; 77: 205-8) to facilitate the patient to contemplate change - i.e. to drink less thereby making reattendance to the Emergency Department (ED) less likely.

Whilst patient consent is necessary for any investigation - for the obtunded patient - the doctor/nurse must act in the patients best interest at the outset - including where judged appropriate to take a BAC - providing feed-back is given at the earliest opportunity when the patient has recovered (Csipke et al, 2007). This is very important as every health care worker has a duty of care to reduce unscheduled hospital reattendance - including that from alcohol misuse (now that it has been proved there is effective treatment of BA with possible BI).

For patients brought to the resuscitation room of an ED it has been shown that the five most common presentations associated with alcohol misuse are collapse, self-abuse, trauma, gastrointestinal bleeding and non-cardiac chest pain. For these conditions a BAC should be requested routinely at the outset (Touquet et al, 2008). To have a simple, robust method which gives an answer of a BAC reading within one minute would be of obvious advantage rather than having to wait for one hour minimum for a laboratory result. Further within that hour events may evolve such that the BAC result, when it returns, is not acted on with the patient being given feed-back when recovered (perhaps due to clinical inertia).

A similar, but even more pressing, situation is in pre-hospital care with Paramedic attendance and the Ambulance Service, with the current increased incidence of collapse from binge/hazardous drinking where, for instance, a low BAC excludes alcohol as a cause of unconsciousness and a raised BAC indicates the need for, at the very least, to protect the airway for the patient made vulnerable by a high BAC (although other pathology e.g. head injury, blood loss or infection may be present as well).

In all of the above situations a stick test for the glucose level using a fingerprick blood sample would routinely be carried out - hence adding BAC to this is the simplest way of potentially improving the standards of care for the patient, whilst highlighting to all that alcohol is a drug that can reduce levels of consciousness and make patients very vulnerable. It has been shown there is poor correlation between clinical signs and BAC level (Cherpitel, Bond J, Ye Y et al. Clinical assessment compared with breathalyser readings in the ER, concordance if ICD10 Y90 and Y91 codes. Emerg Med J 2005;22:689-95), together with the possibility of tolerance to alcohol having developed masking the actual BAC level (Urso et al, 1981). It is increasingly understood that very high BACs may indicate dependency where, not only is BI together with follow-up in the community necessary, but also such patients risk symptoms & signs of withdrawal with delirium tremens a possibility. A very high BAC is an alert for this possibility and the need for added vigilance.

Over fourteen million people a year are treated in emergency departments (EDs) in England(1). In view of the strong association between alcohol misuse and health related problems such as accidental injury and violence, it is not surprising that alcohol misuse is greatly more prevalent among people attending EDs than among the general population. As many as one in three attendees have consumed alcohol immediately before their presentation, and more than two thirds of attendances after midnight may be alcohol related (2).

A recent multi-level analysis of alcohol related injury in ED's in 17 countries, found that up to 46% of injuries were alcohol related for samples as a whole, with this rising to 80% in some countries when considering drinkers only (3). The top ten presenting ED conditions associated with alcohol misuse are fall, collapse, head injury, assault, accident, unwell, non-specific gastrointestinal problems, psychiatric, cardiac and general repeat attends.

It is established practice in some centres to routinely carry out blood alcohol levels, but by laboratory methods (4)(5)(6)(7)(8)(9). However because of not having the facility for 'near patient testing' this generally takes more than one hour largely due to the logistics of returning the result to the requesting department; even if it is transmitted by placing the result on the hospital chemical pathology computer screen for the acute physician to read.

Those practiced in the art will appreciate that the excessive use of alcohol becomes serious when consumption is in excess of the range normally associated with drink driving cases, and traditional breath alcohol meters are not suitable for recording alcohol levels in an emergency medicine environment.Breathalysers are perceived by patients to be judicial and therefore threatening as well as being judgemental. They also suffer from uncertainty as to whether mouth alcohol or alcohol from vomit has affected the result. BAC by finger-prick - like a blood glucose finger-prick test - is perceived as being medical. Only blood alcohol is considered an accurate assessment of the level of alcohol in the blood pharmacologically affecting the patient at the time of recording (rising with further alcohol in the stomach, or falling with the liver metabolising at approximately 20mgs/100ml/hour).

Thus we see that the desirability of testing individuals to determine the extent to which they are under the influence of alcohol is as we have shown already very well known. Alcohol use in most countries is legal, and supply of alcohol is widely available. In other countries where alcohol consumption is prohibited it nevertheless can be consumed illicitly and may still represent a problem to the medical profession.

Many people develop alcohol dependence and tolerance, resulting in a significant proportion of the population having a problem with alcohol intoxication at some time or other. It is therefore highly desirable to test for the level of alcohol intoxication and so this has been the subject of many studies hitherto.

There are many schemes for testing an individual to determine their intoxication from alcohol and they range from physical tests such as requiring them to walk in a straight line, and touching their nose with their eyes shut, to various designs of breathalyzer and alcometer and even more sophisticated laboratory techniques requiring accurately measured reagents and body fluid samples.

Physical tests require the participant to be mobile and willing, and are usually restricted to police checks to judge fitness for driving or other duties; breathalysers and alcometers require the participant to be cooperative, and laboratory techniques require sophisticated non-portable machinery and facilities.

These methods are generally unsuitable for EDs because they either require active participation from the subject although they are probably incapacitated through intoxication or injury, or the methods take too long for a result to be meaningful or incorporated into the treatment given, or else the methods fail to provide accurate results at high alcohol concentrations.

Test strips or sticks are available which are immersed in a sample of urine or saliva, and which include an indicator which changes colour in response to the presence of alcohol and these are well known and constitute a crowded art. However we have shown there are sound clinical arguments why saliva is of little use to the clinician in a hospital environment in determining the alcohol level of a patient who is though to be under the influence of alcohol.

Recent drinking, most particularly in the intervening period of some fifteen minutes from the time the patient was drinking alcohol to the time the patient is admitted to accident and emergency will produce abnormally high levels of salivary alcohol compared to the quantity of alcohol in his or her body. Conversely if the patient has consumed abnormally high levels of alcohol (such as is the current trend in Britain for binge drinking) potentially vast quantities of alcohol will reside in the stomach still to be absorbed but the salivary alcohol will be abnormally and dangerously underestimated because the doctor in attendance will be unaware of the danger to the patient that is to follow. Simple saliva alcohol test strips are of little value here because they have a poor colour response, frequently have to be read in less than favourable lighting conditions and cannot give the sensitivity required to measure rising alcohol levels that may be inferred, but with no way of verifying by a doctor under the constraints of an accident and emergency situation.

Even worse, laboratory tests for alcohol content of blood take time and are of little value in dealing with a patient whose needs may well be immediate.

Alcohol levels may also be detected using urine but this system of analysis requires complicated laboratory instruments and there is questionable value in using urine for this purpose because urine entering the bladder has an ever-reducing level of alcohol and each voiding of the bladder represents an average value of alcohol from the time of the previous voiding to the time of the current voiding but these times are rarely noted.

The unique experiences and professions of the inventors have led them to conclude that the only way that admissions departments and ED's can responsibly deal with the high proportion of admissions patients under the various influence of alcohol is to routinely test the blood alcohol of these people as quickly as possible using portable equipment.

Doctors and nurses are well practiced in the art of obtaining blood from a finger prick test site, or by venous or intramuscular sampling of blood and this is done routinely at hospital for a wide range of tests and screens such as hepatitis, HIV, blood glucose monitoring and the like. Hitherto it has not been appreciated by those practiced in the art that blood alcohol concentrations are essential at the point of admission for such people.

The unique experiences and professions of the inventors have led them to conclude that absolute accuracy of blood alcohol result is not necessary and in any case is a futile activity because the blood level will not be steady state and will either be decreasing (because the patient is metabolizing previous drinking episodes) or else increasing (because the donor is absorbing recently consumed alcohol from the gut). It will be appreciated by those experienced in the art that neither of these situations will yield a satisfactory saliva alcohol reading. What is more essential is a rapid system for detecting the approximate alcohol level in a blood sample with a reasonable test-to-test reproducibility for that particular batch of test devices. This is because blood alcohol correlates accurately with the effect of alcohol on the brain and hence on the limbic system and neurological functions.

What is more essential is a rapid system for detecting the approximate alcohol level in a blood sample with a reasonable test accuracy, so that a patient's progress may be monitored reliably.

The inventors have also discovered that far from high accuracy being futile because blood alcohol levels are dynamic, high accuracy is also considered detrimental by the authorities because some individuals will refuse a blood alcohol test in view of its historical medico-legal associations with police action against motorists and operators of machinery, and the like if it is admissible as evidence. The inventors realize that having a system that is not admissible as evidence because the standard deviation of the results is too high is an advantage in administering the test precisely because there is no risk of police action after the event. The proposed device has a high accuracy at alcohol values close to zero and this reduces to around 5% at 200mgs% and this is ideal for the intended purpose of the instrument, however the instrument has capability of all levels of accuracy and all levels are covered by this patent.

A negative BAC - or a result say less than 80mgs/100ml - is an excluder of a diminished level of consciousness. This is important - e.g. with a patient who has had a head injury who smells of alcohol or where there is a history of drinking - when the BAC is low it is a total excluder of alcohol being a cause, or part cause, of this diminished level of consciousness - heightening the urgency for a CT of the head for possible intracranial bleeding (Quaghebeur G & Richard P. Comatose patients smelling of alcohol. BMJ 1989;299:410)

### SUMMARY OF THE INVENTION

In accordance with one aspect of the present invention there is provided a method of detection of alcohol, the method comprising taking a sample in a sample volume, presenting the sample volume to a delivery path whereby at least a portion of the sample volume is presented to a chamber for vapour equalization with the air therein and the chamber includes means for temperature conditioning the vapour equalization in the chamber between the sample volume and the air there present, the chamber being associated with a fuel cell whereby a present electrical voltage across the fuel cell relative to a developed temperature condition is indicative of alcohol content in the vapour equalization and so the sample volume taken.

In accordance with a further aspect of the present invention there is provided a detection apparatus comprising:
a) a chamber associated with a delivery path for a sample volume;
b) conditioning means for the chamber to temperature condition the sample volume in a vapour equalization with air in a head space for the chamber;
c) a fuel cell associated with the head space to sample the vapour equalization to determine the present electrical voltage in the fuel cell indicative of alcohol level in the vapour equalization relative to a temperature condition.

Typically, the sample volume is blood. Generally, the sample volume is a drop.

Typically, the method and/or apparatus include means to stabilize the temperature condition.

Generally, the delivery path depends upon capillary action. Generally, the delivery path essentially isolates the chamber from a collector. Generally, the collector comprises means for taking blood. Possibly, the means for taking blood comprises a lancet. Possibly, the collector is arranged to be reciprocally shaped with part of the delivery path for transfer therealong of the sample volume. Possibly, the collector includes and/or is coated with a blood anti-clotting agent.

Possibly, the fuel cell has an orifice protruding into the chamber.

Possibly, the temperature conditioning means includes heaters. Generally, the method and/or apparatus includes means for achieving a desired developed temperature condition irrespective of environmental temperature and/or presented sample volume temperature initially as taken.

Typically, the method and/or apparatus includes bellows to draw the sample volume and air in vapour equalization into the fuel cell.

Possibly, the temperature conditioning means has a variable time period dependent upon establishing vapour equalization in the chamber.

Possibly, the method and/or apparatus includes an electrochemical sensor. Possibly, the electrochemical sensor is for blood glucose.

Generally, the method and/or apparatus will include a controller having a plurality of algorithms and/or response profiles for the different temperature conditions equating electrical voltage at each temperature condition to alcohol content in the vapour equalization of the sample volume and air within the chamber.

Preferably the blood sample is placed within the instrument using a disposable device to avoid contamination and health issues. This disposable device is preferably in the form of a swab or receptacle for the blood. It has to provide a sample of sufficient size so that it contains sufficient alcohol to generate an equilibrium in the headspace within the instrument.

In order to carry out a blood alcohol test, a small quantity of blood, preferably a finger-prick sample of blood consisting of one drop is produced preferably by a small fingertip lancet device capable of painlessly piercing the skin to collect fresh blood. Once a hanging droplet of blood is available on the finger the collector is applied to the droplet and blood is absorbed by the collector.

The instrument is turned on and allowed to prepare itself for the analysis. Preferably the instrument displays the number of times it has been used for calibration purposes. Preferably the instrument then goes through a calibration and check process which preferably includes a countdown process. Preferably once the instrument is ready it emits an audible signal to tell the operator to insert the sample. Preferably the collector can be inserted into the instrument without any risk of contamination of the instrument or the operator from the blood. Preferably the collector places the sample within close proximity of the detector in the head space. Preferably the detector consists of a fuel cell sensor such as those produced by Fuel Cell Sensors Limited of 4A Brecon Court, William Brown Close, Llantarnam Park. Cwmbran, NP44 3AB, UK. Preferably the head space has a volume no greater than 1.0ml for a 30 microlitre blood sample. Preferably the instrument has a form of control over temperature in the headspace.

Preferably the instrument has a timer to allow for the sample to equilibrate its alcohol level with the air in the headspace according to universal gas laws. Preferably this timer may be adjusted according to the level of accuracy of result that is required, acknowledging that this analysis is required quickly and that accuracy is improved with time. Preferably this time for equilibration can be adjusted without dismantling the instrument and can be achieved with the minimum of complication.

Preferably the value of blood alcohol is displayed digitally. Preferably the value is displayed for a short period of time thereafter to permit the operator to record it. Preferably this value is stored into memory and can be recovered later even if it was not written down at the time of the actual analysis.

Preferably the collector may then be easily removed and discarded or disposed of in clinical waste.

Preferably the instrument is designed so that it purges itself ready for the next analysis operation.

Preferably the instrument is used in conjunction with other medical tests that use a blood sample, such as glucose metering or blood gas analysis and detection. For example the circuitry and sample receptacle necessary for blood alcohol monitoring are suitable for incorporating into fingerprick blood sampling for glucose monitoring so that both analyses may be carried out simultaneously.

### PRIOR ART

Personal blood alcohol level testing kit.
Inventor: TITMAS TED (US)
EC: C12Q1/26; C12Q1/28; (+1)
Publication info: US5563073 - 1996-10-08
This patent covers a saliva alcohol test device arrangement.

Test watch for determining the level of alcohol in the blood
Inventor: XAVIER ABOULKER
Applicant: ABOULKER XAVIER (FR)
EC: G01N33/98; G04B47/06
IPC: G01 N33/98; G04B47/06; G01 N33/98 (+4)
Publication info: FR2709573 - 1995-03-10
This patent covers a watch device that can be used as a breathalyser

Blood alcohol test appts.
Inventor: KOUKAL ANTON (DE)
Applicant: KOUKAL ANTON (DE)
EC: G01N33/497A; G01N33/98
IPC: G01N33/497; G01N33/98; G01N33/483 (+5)
Publication info: DE4300029 - 1994-07-07
This patent covers a breathalyzer device

Electronic monitor of blood alcohol and speed for a motor vehicle
Inventor: / Applicant: JOSE DA ENCARNACAO FRANCO (PT)
IPC: B60K28/06; B60K31/00; B60K31/02 (+9)
Publication info: PT101176 - 1994-09-30
This patent covers a breathalyzer device fitted to vehicles.

Blood alcohol monitor
Inventor: PHILLIPS MARY F (US); HAWTHORNE JEFFREY S (US)
Applicant: SAFETY TECH PARTNERS LTD (US); PHILLIPS MARY F (US); (+1) EC: G01 N33/98
IPC: G01N33/98; G01N33/98; (IPC1-7): A61B5/00
Publication info: WO9407407 - 1994-04-14
This patent covers a device to measure alcohol expelled through a person's skin

Blood alcohol monitor
Inventor: PHILLIPS MARY F (US); HAWTHORNE JEFFREY S (US)
Applicant: SAFETY TECH PARTNERS LTD (US)
EC: G01N33/497A
IPC: G01N33/497; G01N33/483; (IPC1-7): A61B5/00
Publication info: US5220919 - 1993-06-22
This patent covers a device to measure alcohol expelled through a person's skin at regular intervals.

Color control system
Inventor: PALMER JOHN L (US); TIMMERMAN MARSHA W (US)
Applicant: ENZYMATICS INC (US)
EC: C12Q1/00F; C12Q1/32; (+1)
IPC: C12Q1/00; C12Q1/32; G01 N33/52 (+6)
Publication info: US5032506 - 1991-07-16
This patent covers the use of diaphorase or lipoamide dehydrogenase to expand the range of alcohol determinations

Apparatus for testing the blood alcohol content
Inventor: STERNAL HANS-JOACHIM (DE)
Applicant: STERNAL HANS JOACHIM
EC: G01 N33/98
IPC: G01 N33/98; G01 N33/98; (IPC1-7): G01 N33/48
Publication info: DE3418373 - 1985-11-21
This patent covers a device to measure alcohol in respiratory air

Determination of alcohol content in blood
Inventor: ALBARDA SCATO
Applicant: DRAEGERWERK AG
EC: G01N33/497A
IPC: G01N33/98; G01N33/49; G01N33/497 (+4)
Publication info: CA1118612 - 1982-02-23
This patent covers a device to measure alcohol in exhaled air

Blood alcohol analyzer
Inventor: KOCH ROBERT B; SKOGEN JOHN D
Applicant: HONEYWELL INC
EC: C12Q1/32
IPC: C12Q1/32; C12Q1/32; (IPC1-7): C12K1/04 (+1)
Publication info: US3941659 - 1976-03-02
This patent covers a fluorescence spectrophotometer machine to measure blood alcohol

Stable blood detecting composition
Inventor: / Applicant: MILES LAB
EC: G01N33/72B4; G01N33/72B6
IPC: G01 N33/72; G01 N33/72
Publication info: GB893318 - 1962-04-04
This patent covers a device to measure occult blood in bodily fluids.

Novel amino alcohol dehydrogenase, method for producing said enzyme, and use of said enzyme
Inventor: ITO NOBUYA (JP); MATSUYAMA AKINOBU (JP); (+1)
Applicant: DAICEL CHEM (US)
EC: C12N9/02B; C12N9/04
IPC: C12N9/02; C12N9/04; C12N9/02 (+4)
Publication info: US2003027306 - 2003-02-06
This patent covers a range of Amino acid dehydrogenases, amine dehydrogenases, and aminotransferases to initiate chemical reactions.

Hitherto no applicants have addressed the issue of blood alcohol, other than using complicated laboratory instrumentation or the detection of alcohol in other media, such as breath, and conversion by calculation to blood alcohol levels.

### FIGURES OF THE PREFERRED EMBODIMENTS

FIG. 1 shows a schematic arrangement of the device and its collector
FIG. 2 shows a schematic arrangement of a collector in place adjacent to the detector.
FIG. 3 shows the controls and graphical interface for the device.

### DESCRIPTION OF THE FIGURES AND OPERATION OF THE DEVICE

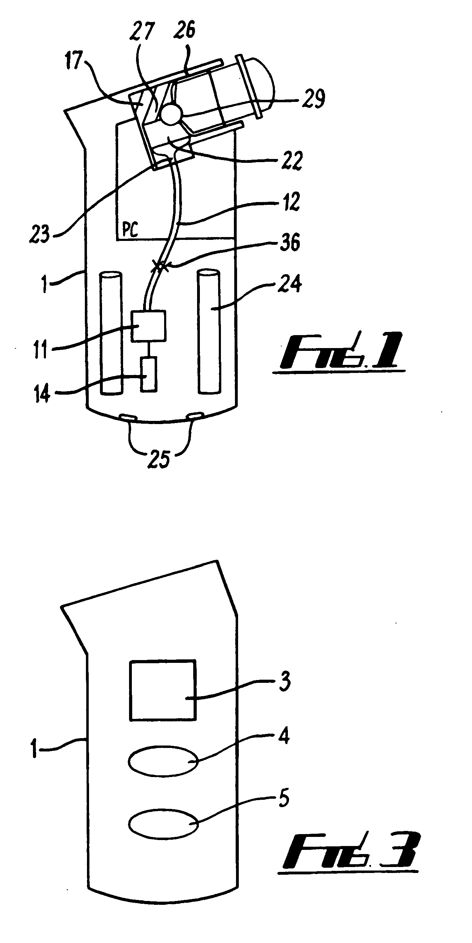

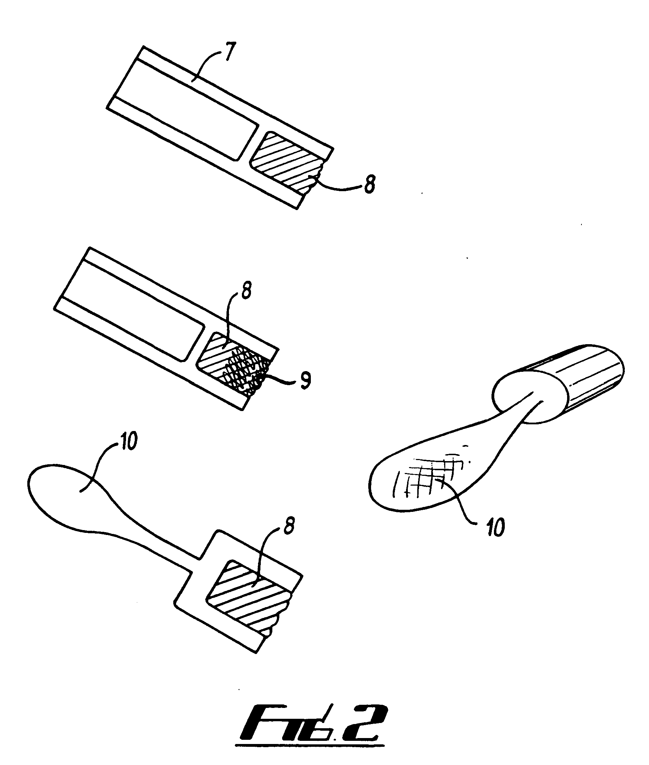

The instrument case (1) (Figure 1) preferably made from impact resistant plastic such as ABS houses a display panel (3) and control switches such as an on/off button (4) and a function button (5). A collector device (7) consists of a moulding made preferably from plastic such as polystyrene, with a recess at one end into which is placed an inert, absorbent material such as glassfibre filter paper or the like (8). The collector may be constructed with hollow tube to minimise the amount of plastic used, or it may conveniently be produced with a handle attached (10), all as shown in Fig 2. Preferably the collector contains an anti-clotting agent such as ethylenediaminetetraacetic acid (EDTA) or sodium fluoride, to prevent the sample from clotting and skinning over.

The instrument houses a fuel cell (22) held within a chamber (17) having a known, small volume such as 1 millilitre, and the input to the fuel cell consists of a very small orifice (27) protruding into the chamber. The chamber has an opening (30) into which the collector fits snugly, such that the absorbent end (8) is about 3mm away from the entrance to the fuel cell (27). Heaters (26) control the temperature of the collector and the environment within the chamber (17) and a thermocouple (29) controls the temperature of the chamber.

The other end of the fuel cell has a small orifice (23) connected to a plastic tube (12) which is attached to bellows unit (11). The electrical actuator (14) is connected to the moving piston of the bellows unit (11). The plastic housing (1) has compartments for batteries (14) which in this illustration are two size AA batteries which may be disposable or rechargeable batteries. Where a rechargeable unit is preferred, contacts (25) allow the unit to be placed in a cradle for convenient storage when the unit will automatically be recharged.

The instrument may be set to accommodate the particular conditions required by the operator, using the push buttons (4) and (5) to toggle through the programme. For example pushing the on/off button turns the instrument on then if button (5) is pushed within three seconds this puts the instrument into set up mode. Button (5) may be used to toggle through the programme for testing time, display units, (for example milligrams/100 ml, promiles and the like). whether the instrument should be back-lit, and whether the instrument should provide a torch facility in situations (such as a road accident) where visibility may be poor. In accident situations a short conditioning time may be chosen since speed is more important than absolute accuracy, while in a hospital environment or where a patient's alcohol level is being monitored periodically, absolute accuracy may be more important than speed of analysis, in which case a longer conditioning time may be chosen. Once the chosen option is displayed on the panel (3) the start button (4) selects that option. After the chosen options, or if button (5) is not pressed, the instrument goes through a conditioning stage of purging and heating in preparation for the collection of a sample. If no action is taken during the toggle, the instrument will turn off automatically after a period of inactivity, such as after 30 seconds.

To collect a sample, a donor's finger is chosen and a standard 30 microlitre lancet is used to draw blood. This activity is common in medical applications and is similar to that used to collect blood for a glucose determination. Once a hanging droplet of blood is available, collector (7) is applied to the finger and the blood is absorbed onto pad (8).

The instrument is turned on and undergoes a countdown period to prepare the instrument. The collector is then pushed into the chamber (17) and the instrument starts its conditioning period. This activates the heater (26) controlled by its thermocouple (29) and starts a timer which continues for the pre-set conditioning time. Experimentation has shown that 45 seconds is sufficient for an approximate analysis (such as at an accident scene) while up to 3 minutes provides a more accurate analysis. During conditioning the alcohol in the blood outgases and develops an equilibrium with the alcohol in the chamber, according to universal gas laws at the temperature given by the heater (26) controlled by its thermocouple (29). This alcohol is prevented from entering the fuel cell (22) by virtue of the small orifice (27) and the volume of air already within the fuel cell, thereby minimising diffusion of conditioned air into the fuel cell until the instrument is ready to read the headspace air.

Once the conditioning time has elapsed, the timer activates solenoid (14) drawing air from tube (12) into the bellows unit, which simultaneously displaces conditioned air from the headspace chamber (17) into the fuel cell (22) so that the fuel cell senses the level of alcohol in the conditioned air. This generates an electrical voltage which is proportional to the quantity of alcohol in the conditioned air sample. The electronics within the unit interpolates the alcohol value from a calibration curve and displays it on the instrument display panel (3).

### EMBODIMENTS

The purpose of the heater (26) is to provide stable conditions for the analysis under all practical situations, because it is envisaged the unit could even be employed in sub-zero conditions such as at road accidents in Scandinavia and the like. The partitioning of alcohol into the headspace is affected considerably by temperature and so it is necessary to be aware of the temperature at the time of the test. In one embodiment of the test it is foreseen that the instrument could be used substantially at ambient room temperature (such as in a hospital emergency room). In this case the instrument may be equipped with sets of calibration data at a range of temperatures. The instrument thermocouple (29) or similar sensing device allows the instrument to choose the appropriate calibration curve for that temperature and provide an accurate alcohol result at a range of temperatures. In this case the heater system may be dispensed with.

In another embodiment the system collector incorporates an electrochemical sensor for glucose so that the instrument may display blood glucose and blood alcohol simultaneously. There are distinct advantages in some emergency medicinal situations (such as coma or collapse) to have readings of both blood glucose and blood alcohol simultaneously. The technology relating to blood glucose is already well established and suitable for incorporation through licensing or independent development.

In another embodiment the alcohol detection system here described may be incorporated into automatic detections systems such as at blood transfusion centres, and routine blood monitoring systems within a hospital or a drug and alcohol rehabilitation clinic, or in locations such as intensive care units, critical care or cardiovascular units where consumption of alcohol is not permitted.

A further embodiment permits the instrument to be used for detection of alcohol in a natural headspace such as exists above grain silos, fermentation vessels, food stores, and the like. Here the instrument is fitted with a fine aspiration needle preferably with a pointed tip, so that it is able to penetrate a septum provided into the vessel or container for the purpose of inserting the needle. The instrument then collects a sample from the headspace by operating the solenoid (14) several times so that conditioned air from the headspace is drawn into the fuel cell (22) aided by the one-way valve (36) in the tube (12) ensuring only one way flow as the bellows (11) cycles. Detection of alcohol in such circumstances may be valuable in early detection of process difficulties such as yeast attack of foodstuffs, or monitoring of a fermentation process.

### ADVANTAGES

The present invention has a number of advantages. These advantages will be apparent in the following description.

By way of example, the present invention is advantageous because blood alcohol accurately represents the state of the patient because it is free from the disadvantages of saliva, breath or urine which can be vastly different from the level of alcohol in the brain and other organs for reasons discussed previously.

By way of further example the present invention will be advantageous because the level of blood alcohol will be obtained from any patient whether conscious, incapacitated, comatose, injured, cooperative or non co-operative via a simple fingerprick blood sample.

By way of further example, the present invention is advantageous because the medical profession prefer to rely on blood alcohol levels over any other medium such as breath or saliva because blood alcohol relates directly to the effects of alcohol on the brain and other organs.

By way of further example, the present invention is advantageous because the medical profession routinely obtain and handle blood samples for a range of other medical tests and are conversant and readily prepared in their areas such as ED's for the taking and handling of blood samples.

By way of further example, the present invention is advantageous because the quantity of blood required is very small being typically one drop, and this quantity is readily obtained by a number of medical lancet devices already used routinely in healthcare, such devices requiring little or no training and virtually painless in use.

By way of further example, the present invention is advantageous because blood alcohol measurements can easily be repeated from time to time as necessary to monitor alcohol levels and avoid the risk of alcohol poisoning from alcohol still remaining in the stomach of an incapacitated patient.

By way of further example, the present invention is advantageous because the test device will also detect the simpler homologue methanol (from methylated spirit) which is toxic and which is often implicated in alcoholics living on the street who frequently require medical attention in ED's.

By way of further example, the present invention is advantageous because the blood alcohol level may be incorporated into other devices separately or simultaneously, for example detection of blood glucose and blood alcohol simultaneously.

By way of further example, the present invention is advantageous because the blood alcohol result is achieved in such a way that it is unlikely to be admissible as evidence, thus preventing patients from refusing a test in case they are subjected to legal action.

## Claims

1. A method of detection of alcohol, the method comprising absorbing a sample onto a collector device, the collector device comprising an absorbent material and a blood anti-clotting agent, the sample having a sample volume, presenting the sample volume to a delivery path whereby at least a portion of the sample volume is presented to a chamber for vapour equalization with the air therein and the chamber includes means for temperature conditioning the vapour equalization in the chamber between the sample volume and the air there present, the chamber being associated with a fuel cell whereby a voltage across the fuel cell relative to a developed temperature condition is indicative of alcohol content in the vapour equalization chamber and to the alcohol content in the sample volume taken, **characterised in that** the sample volume comprises blood.

2. A method according to claim 1, **characterised in that** the sample volume is a drop.

3. A method according to any of the preceding claims, **characterised in that** the method includes stabilising the temperature condition.

4. A method according to any of the preceding claims, **characterised in that** the blood sample is placed within the collector using a disposable device to avoid contamination and health issues, which disposable device may be in the form of a swab or receptacle for the blood,

5. A method according to any of the preceding claims, **characterised in that** the instrument is turned on and allowed to prepare itself for the analysis, and may then display the number of times it has been used for calibration purposes, and having been turned on, the instrument may go through a calibration and check process, including a countdown process.

6. A method according to any of the preceding claims, **characterised in that** the blood alcohol value is displayed for a short period of time to permit the operator to record it, and may be stored into memory such that the blood alcohol value can be recovered later even if it was not written down at the time of the actual analysis,

7. A method according to any of the preceding claims, **characterised in that** the instrument is used in conjunction with other medical tests that use a blood sample, such as glucose metering or blood gas analysis and detection.

8. A detection apparatus comprising:
a) a collector device, the collector device comprising an absorbent material and a blood anti-clotting agent;
b) a chamber associated with a delivery path for a sample volume;
c) conditioning means for the chamber to temperature condition the sample volume in a vapour equalization with air in a head space for the chamber;
d) a fuel cell associated with the head space to sample the vapour equalization to determine the voltage in the fuel cell indicative of alcohol level in the vapour equalization relative to a temperature condition, **characterised in that** the sample volume is blood.

9. Apparatus according to claim 8, **characterised in that** the apparatus includes means to stabilize the temperature condition.

10. Apparatus according to claims 8 or 9, **characterised in that** the delivery path essentially isolates the chamber from the collector, which collector may comprise means for taking blood, and may comprise a lancet.

11. Apparatus according to any of claims 8 to 10, **characterised in that** the fuel cell has an orifice protruding into the chamber.

12. Apparatus according to any of claims 8 to 11, **characterised in that** the temperature conditioning means has a variable time period dependent upon establishing vapour equalization in the chamber, which time period may be adjusted according to the level of accuracy of result that is required, acknowledging that this analysis is required quickly and that accuracy is improved with time.

13. Apparatus according to any of claims 8 to 12, **characterised in that** the apparatus includes an electrochemical sensor, which sensor may be for blood glucose, and the apparatus may include a fuel cell sensor.

14. Apparatus according to any of claims 8 to 13, **characterised in that** the apparatus has a form of control over temperature in the headspace and may have a timer to allow for the sample to equilibrate its alcohol level with the air in the headspace according to universal gas laws,

15. Apparatus according to any of claims 8 to 14, **characterised in that** the apparatus is suitable for performing other medical tests that use a blood sample, such as glucose metering or blood gas analysis and detection, and the circuitry and sample receptacle necessary for blood alcohol monitoring are suitable for incorporating into fingerprick blood sampling for glucose monitoring so that both analyses may be carried out simultaneously.

## Patentansprüche

1. Verfahren zum Erfassen von Alkohol, wobei das Verfahren die folgenden Schritte aufweist:
Absorbieren einer Probe auf einer Kollektorvorrichtung,
welche ein absorbierendes Material sowie ein Anti-Blutgerinnungsmittel aufweist, wobei die Probe ein Probenvolumen hat;
Darbieten des Probenvolumens an einem Zuführungsweg, wobei zumindest ein Teil des Probenvolumens einer Kammer zum Dampfausgleich mit der darin enthaltenen Luft zugeführt wird und die Kammer ein Mittel zur Temperaturkonditionierung des Dampfausgleichs in der Kammer zwischen dem Probenvolumen und der dort vorhandenen Luft enthält, wobei die Kammer einer Brennstoffzelle zugeordnet ist und wobei eine Spannung an der Brennstoffzelle relativ zu einem entwickelten Temperaturzustand einen Hinweis auf den Alkoholgehalt in der Dampfausgleichskammer sowie auf den Alkoholgehalt in dem entnommenen Probenvolumen darstellt, **dadurch gekennzeichnet, dass** das Probenvolumen Blut aufweist.

2. Verfahren nach Anspruch 1, **dadurch gekennzeichnet, dass** das Probenvolumen ein Tropfen ist.

3. Verfahren nach einem der vorhergehenden Ansprüche, **dadurch gekennzeichnet, dass** das Verfahren ein Stabilisieren des Temperaturzustands enthält.

4. Verfahren nach einem der vorhergehenden Ansprüche, **dadurch gekennzeichnet, dass** die Blutprobe innerhalb des Kollektors unter Verwendung einer Einwegvorrichtung platziert wird, um eine Kontamination und Gesundheitsprobleme zu vermeiden, wobei die Einwegvorrichtung in Form eines Tupfers oder eines Behälters für das Blut vorliegt.

5. Verfahren nach einem der vorhergehenden Ansprüche, **dadurch gekennzeichnet, dass** man das Instrument einschaltet und ihm gestattet, sich auf die Analyse vorzubereiten, und es dann anzeigen kann, wie oft es für Kalibrierungszwecke benutzt worden ist, und wobei nach dem Einschalten das eingeschaltete Instrument einen Kalibrierungs- und Prüfvorgang einschließlich eines Rückwärtszählvorgangs durchlaufen kann.

6. Verfahren nach einem der vorhergehenden Ansprüche, **dadurch gekennzeichnet, dass** der Blutalkoholwert für eine kurze zeitdauer angezeigt wird, um dem Bediener zu ermöglichen, ihn aufzuzeichnen, und in einem Speicher derart abgespeichert wird, dass der Blutalkoholwert später wiederhergestellt werden kann, und zwar auch dann, wenn er zum Zeitpunkt der tatsächlichen Analyse nicht aufgeschrieben wurde.

7. Verfahren nach einem der vorhergehenden Ansprüche, **dadurch gekennzeichnet, dass** das Instrument zusammen mit anderen medizinischen Tests verwendet wird, welche eine Blutprobe verwenden, wie z.B. die Messung von Glukose oder die Analyse von Blutgas bzw. die entsprechende Erfassung.

8. Erfassungsgerät, welches aufweist:
a) eine Kollektorvorrichtung, welche ein absorbierendes Material sowie ein Anti-Blutgerinnungsmittel aufweist;
b) eine Kammer, die einem Zuführungsweg für ein Probenvolumen zugeordnet ist;
c) ein Konditionierungsmittel für die Kammer zum Temperaturkonditionieren des Probenvolumens bei einem Dampfausgleich mit Luft in einem Kopfvolumen für die Kammer;
d) eine dem Kopfvolumen zugeordnete Brennstoffzelle zur Probennahme beim Dampfausgleich zum Bestimmen der Spannung in der Brennstoffzelle, welche einen Hinweis auf den Alkoholgehalt beim Dampfausgleich relativ zu einem Temperaturzustand darstellt, **dadurch gekennzeichnet, dass** das Probenvolumen Blut ist.

9. Gerät nach Anspruch 8, **dadurch gekennzeichnet, dass** das Gerät ein Mittel zum Stabilisieren des Temperaturzustands enthält.

10. Gerät nach Anspruch 8 oder 9, **dadurch gekennzeichnet, dass** der Zuführungsweg im wesentlichen die Kammer von dem Kollektor isoliert, wobei der Kollektor ein Mittel zur Blutentnahme aufweisen kann und eine Lanzette aufweisen kann.

11. Gerät nach einem der Ansprüche 8 bis 10, **dadurch gekennzeichnet, dass** die Brennstoffzelle eine in die Kammer ragende Öffnung hat.

12. Gerät nach einem der Ansprüche 8 bis 11, **dadurch gekennzeichnet, dass** das Temperaturkonditionierungsmittel eine variable zeitdauer hat, die von der Herstellung des Dampfausgleichs in der Kammer abhängt, wobei die Zeitdauer entsprechend dem Grad der gewünschten Genauigkeit des Ergebnisses eingestellt werden kann, und wobei eingeräumt wird, dass diese Analyse rasch benötigt wird und dass die Genauigkeit mit der Zeit verbessert wird.

13. Gerät nach einem der Ansprüche 8 bis 12, **dadurch gekennzeichnet, dass** das Gerät einen elektrochemischen Sensor enthält, welcher ein Sensor für Blutglukose sein kann, und dass das Gerät einen Brennstoffzellensensor enthalten kann.

14. Gerät nach einem der Ansprüche 8 bis 13, **dadurch gekennzeichnet, dass** das Gerät eine Form der Steuerung über die Temperatur in dem Kopfvolumen hat und einen Zeitgeber haben kann, der es der Probe gestattet, ihren Alkoholgehalt mit der Luft in dem Kopfvolumen gemäß der universellen Gasgesetze ins Gleichgewicht zu bringen.

15. Gerät nach einem der Ansprüche 8 bis 14, **dadurch gekennzeichnet, dass** sich das Gerät zur Durchführung anderer medizinischer Tests eignet, welche eine Blutprobe verwenden, wie z.B. die Messung von Glukose oder die Analyse von Blutgas und die entsprechende Erfassung, und dass die Schaltkreise und der Probenbehälter, welche für die Blutalkoholüberwachung notwendig sind, sich für den Einbau in eine Blutprobennahme durch Fingerpieksen für die Glukoseüberwachung eignen, so dass beide Analysen gleichzeitig durchgeführt werden können.

## Revendications

1. Procédé de détection d'alcool, le procédé comprenant l'absorption d'un échantillon sur un dispositif collecteur, le dispositif collecteur comprenant un matériau absorbant et un agent anticoagulant du sang, l'échantillon ayant un volume d'échantillon; la présentation du volume d'échantillon à un canal de transport permettant ainsi la présentation d'au moins une partie du volume d'échantillon à une enceinte pour une égalisation de pression de vapeur avec l'air s'y trouvant, l'enceinte comprenant un moyen de conditionnement en température de l'égalisation de pression de vapeur dans l'enceinte entre le volume d'échantillon et l'air y étant présent, l'enceinte étant associée à une pile à combustible moyennant laquelle une tension dans la pile à combustible relativement à une condition de température développée indique une teneur en alcool dans l'enceinte d'égalisation de pression de vapeur et à la teneur en alcool dans le volume d'échantillon prélevé, **caractérisé en ce que** le volume d'échantillon comprend du sang.

2. Procédé selon la revendication 1, **caractérisé en ce que** le volume d'échantillon est une goutte.

3. Procédé selon l'une quelconque des revendications précédentes, **caractérisé en ce que** le procédé comprend la stabilisation de la condition de température.

4. Procédé selon l'une quelconque des revendications précédentes, **caractérisé en ce que** l'échantillon de sang est placé à l'intérieur du collecteur en utilisant un dispositif jetable pour éviter les problèmes de contamination et les problèmes sanitaires, ledit dispositif jetable pouvant se trouver sous la forme d'un tampon ou d'un réceptacle pour le sang.

5. Procédé selon l'une quelconque des revendications précédentes, **caractérisé en ce que** l'instrument peut se préparer à l'analyse lorsqu'il est activé, et peut alors afficher le nombre de fois où il a été utilisé à des fins d'étalonnage, et après son activation, l'instrument peut subir un processus d'étalonnage et de vérification, y compris un processus de compte à rebours.

6. Procédé selon l'une quelconque des revendications précédentes, **caractérisé en ce que** la valeur d'alcoolémie est affichée pendant une courte durée pour permettre à l'opérateur de l'enregistrer, et peut être stockée en mémoire de manière à ce que la valeur d'alcoolémie puisse être récupérée ultérieurement même si elle n'a pas été consignée au moment de l'analyse elle-même.

7. Procédé selon l'une quelconque des revendications précédentes, **caractérisé en ce que** l'instrument est utilisé conjointement à d'autres tests médicaux qui utilisent un échantillon de sang, tels qu'une mesure de la glycémie ou une analyse et une détection des gaz du sang.

8. Appareil de détection, comprenant :
a) un dispositif collecteur, le dispositif collecteur comprenant un matériau absorbant et un agent anticoagulant du sang ;
b) une enceinte associée à un canal de transport pour un volume d'échantillon ;
c) des moyens de conditionnement pour l'enceinte permettant un conditionnement en température du volume d'échantillon en égalisation de pression de vapeur avec l'air dans un espace de tête de l'enceinte ;
d) une pile à combustible associée à l'espace de tête pour échantillonner l'égalisation de pression de vapeur afin de déterminer la tension dans la pile à combustible indiquant un niveau d'alcool dans l'égalisation de pression de vapeur relativement à une condition de température, **caractérisé en ce que** le volume d'échantillon est du sang.

9. Appareil selon la revendication 8, **caractérisé en ce que** l'appareil comprend un moyen de stabilisation de la condition de température.

10. Appareil selon la revendication 8 ou 9, **caractérisé en ce que** le canal de transport isole sensiblement l'enceinte du collecteur, ledit collecteur pouvant comprendre un moyen de prélèvement de sang, et pouvant comprendre une lancette.

11. Appareil selon l'une quelconque des revendications 8 à 10, **caractérisé en ce que** la pile à combustible possède un orifice en saillie dans l'enceinte.

12. Appareil selon l'une quelconque des revendications 8 à 11, **caractérisé en ce que** le moyen de conditionnement en température a une durée d'action variable dépendant de l'établissement de l'égalisation de pression de vapeur dans l'enceinte, ladite durée pouvant être ajustée selon le niveau de précision requis pour le résultat, en reconnaissant que cette analyse doit être rapide et que la précision s'améliore avec le temps.

13. Appareil selon l'une quelconque des revendications 8 à 12, **caractérisé en ce que** l'appareil comprend un capteur électrochimique, ledit capteur pouvant concerner la glycémie, et l'appareil peut comprendre un détecteur de pile à combustible.

14. Appareil selon l'une quelconque des revendications 8 à 13, **caractérisé en ce que** l'appareil a une forme de régulation de la température dans l'espace de tête et peut avoir un minuteur pour permettre à l'échantillon d'équilibrer son niveau d'alcool avec l'air dans l'espace de tête en fonction des lois universelles des gaz.

15. Appareil selon l'une quelconque des revendications 8 à 14, **caractérisé en ce que** l'appareil est adapté pour réaliser d'autres tests médicaux qui utilisent un échantillon de sang, tels qu'un suivi de la glycémie ou une analyse et une détection des gaz du sang, et **en ce que** le circuit ainsi que le réceptacle de l'échantillon nécessaires pour le suivi de l'alcoolémie sont adaptés pour être incorporés dans un lecteur de glycémie avec prélèvement d'une goutte de sang par piqûre au bout d'un doigt de manière à ce que les deux analyses puissent être réalisées simultanément.
